# EUROPEAN PATENT SPECIFICATION

(11) **EP 3 046 515 B1**
(45) Date of publication and mention of the grant of the patent: **07.03.2018**
(21) Application number: 14759183.8
(22) Date of filing: 04.09.2014
(51) Int. Cl.: A61F 2/915, A61F 2/88

(54) **INTRALUMINAL ENDOPROSTHESIS WITH OPTIMIZED ACTIVE INGREDIENT DISTRIBUTION**
INTRALUMINALE ENDOPROTHESE MIT OPTIMIERTER WIRKSTOFFVERTEILUNG
ENDOPROTHÈSE INTRALUMINALE DOTÉE D'UNE DISTRIBUTION OPTIMISÉE DU PRINCIPE ACTIF

(30) Priority: 17.09.2013 EP 13184744
(43) Date of publication of application: 27.07.2016
(73) Proprietor: Cortronik GmbH, 18119 Rostock-Warnemünde (DE)
(72) Inventor: STIEHM, Michael, 18057 Rostock (DE); LOOTZ, Daniel, 18119 Rostock (DE); LEDER, Alfred, 18209 Wittenbeck (DE); BREDE, Martin, 18059 Rostock (DE)
(74) Representative: Galander, Marcus
(86) International application number: PCT/EP2014/068855
(87) International publication number: WO 2015/039880

(56) References cited:
- EP-A2- 2 016 960
- EP-A2- 2 491 965
- WO-A1-2010/138726
- US-A1- 2007 055 352

## Description

The present invention relates to an intraluminal endoprosthesis suitable for implantation in a bodily vessel through which a medium flows and having a cylindrical main body with a first end and a second end, wherein the main body consists of a main structure, which is suitable for attaining a mechanical supporting effect in the bodily vessel.

Within the scope of this invention, an intraluminal endoprosthesis is understood to mean all implants which are suitable for implantation in a bodily vessel. In particular, these are stents, which are usually introduced by means of catheter technology into bodily vessels (hollow organs, in particular blood vessels). Endoprostheses of this type are in particular implanted in the bodily vessels in order to hold these open. For example, these may be blood vessels after an angioplasty. Furthermore, endoprostheses of this type are also used in cancer treatment in order to hold open airways, bile ducts or the esophagus, after constriction caused by malignant tumors.

The invention will be described hereinafter on the basis of a stent as is used in the treatment of constrictions in arterial blood vessels. The present invention is suitable for this application, but is not limited thereto.

The constrictions of arterial blood vessels referred to as stenoses are usually caused by arteriosclerosis. Here, blood fats, thrombi, connective tissue and, to a lesser extent, also lime deposit in the vessel walls. These harden and narrow the blood vessels. Constrictions of this type can be treated with what is known as angioplasty. Here, a balloon is guided by means of catheter technology to the point of the constriction, is acted on by a fluid and is expanded, and the constriction is thus widened again. In order to keep the vessel open permanently, a stent is implanted at the point of the constriction.

A stent of this type consists of a substantially cylindrical main body through which a fluid can flow. This main body is formed by a main structure, which has an open structure and is suitable for exerting a supporting effect on the vessel.

Within the scope of the invention, a main structure of an intraluminal endoprosthesis is understood to mean an open structure that exerts a mechanical supporting effect on the bodily vessel.

However, when implanting a stent in the manner described above, there is always the risk of thrombus formation or restenosis. The stent, as an implanted foreign body, induces local blood clotting, and therefore a thrombus may form in the stent. A constriction of the stent, or what is known as restenosis, may also occur due to the new formation of binding tissue.

In order to reduce the risk of thrombus formation and restenosis, drug-coated stents or what are known as drug eluting stents are often used in the prior art. Stents of this type are either coated directly with the active ingredient, have reservoirs containing the active ingredient and/or have a coating that contains the active ingredient and releases this in a manner controlled over time.

EP 2016960 A2 describes a method for the production of an active substance depot, which is formed for the mechanical connection with a surface of an endovascular implantable body, comprises (a) providing one or more polymers, (b) providing one or more active substances and (c) producing an active substance depot from the polymers and the active substances, where the active substance depot is formed in such a way that it is mechanically connectable by force effect and/or adhesive application with the surface of the body.

US 2007/0055352 A1 describes a medical device for delivering a therapeutic agent to a body lumen. The device may generally be a stent with an inner sidewall surface and an outer sidewall surface, with a plurality of struts having a plurality of openings therein. An inner and outer layer may be applied to the stent, forming pockets within at least a portion of the openings. The pockets may be filled with at least one therapeutic agent. The pockets may take a variety of shapes and sizes, and may be designed for release or rupture in variety of ways.

EP 2491965 A2 describes an implant comprising a hollow cylindrical main structure with continuous openings and a coating, which releases a pharmaceutically active substance, where a cross-sectional area (50%) and the continuous openings of a predetermined section having a smallest cross-sectional area are covered in a dilated state with the coating that releases the pharmaceutically active substance. Each continuous opening is contained in a closed cell of the main structure, where a cell is formed by segments that are bent and/or curved. The pharmaceutically active substance is predominantly released from the coating in an abluminal direction. The coating comprises layers, where a first layer is located in the luminal direction and is a diffusion barrier for the pharmaceutically active substance.

WO 2010/138726 A1 provides apparatus and methods for treating tissue by delivering at least one therapeutic agent to the tissue. In one embodiment, the apparatus comprises first and second membranes in sealing engagement with strut segments of at least one stent. A first membrane pocket is disposed between the first and second membranes, and a first therapeutic agent is disposed within the first membrane pocket. In other embodiments, the first and second membranes may be disposed between first and second spaced apart stents, or only a single membrane may be provided. In each instance, the quantity of the therapeutic agent delivered is not limited by the surface area of the stent struts or the lumen diameter of the struts

The object of the present invention is to provide an intraluminal endoprosthesis that carries an active ingredient and releases this as slowly as possible in a manner controlled over time at the site of implantation of the intraluminal endoprosthesis. In particular, a drug eluting stent is to be provided that minimizes the risk of thrombus formation and restenosis by means of local active ingredient release at the site of implantation.

The stated problem is solved by an endoprosthesis having the combination of features of independent claim 1. Advantageous embodiments of the endoprosthesis according to the invention are specified in the dependent claims.
In accordance with the invention, at least one additional carrier structure is connected to the main structure, wherein the main structure and carrier structure are formed integrally, wherein the carrier structure carries at least one therapeutic active ingredient, made of the same material as the main structure and connected to the main structure in such a way that the carrier structure is located in an area of abated flow when a fluid passes through the main body from the first end thereof in the direction of the second end thereof. In accordance with the fundamental concept of the invention, the main structure and carrier structure of the endoprosthesis is constructed in such a way that, as a fluid flows through the endoprosthesis from the first to the second end, areas of abated flow and recirculation areas of the flow form where the carrier structure is located. The endoprosthesis is then implanted in such a way that the carrier structure carrying the active ingredient is located directly at the tissue to be treated.
An active ingredient in the sense according to the invention is understood to mean a drug with pharmaceutical effect that is used in the human or animal body for healing, relief, prevention or identification of diseases. Active ingredients in particular comprise Paclitaxel, Sirolimus and derivatives thereof. In particular, active ingredients that act on mTOR are advantageous, as well as RAS inhibitors, particularly those that prevent RAS adhesion.
The concept forming the basis of the present invention is that of connecting the endoprosthesis to an additional carrier structure carrying the active ingredient. The main structure and in particular the carrier structure form areas of abated flow, in which the active ingredient is released. The carrier structure is connected to the main structure and is arranged such that it is located in an area of abated flow. Due to the arrangement according to the invention of main structure and carrier structure with the active ingredient and the resultant release of the active ingredient in an area of abated flow, the active ingredient is not transported away from the site of implantation by the bodily fluid (for example blood). A higher active ingredient concentration at the site of implantation is thus achieved, and the active ingredient is prevented from being distributed and accumulating in the entire organism.

The carrier structure differs functionally and mechanically from the main structure. The main structure of the endoprosthesis exerts a mechanical supporting effect on the vessel wall of the hollow organ. The carrier structure serves as a physical carrier for the active ingredient and does not contribute to, or hardly contributes to, the mechanical supporting effect of the endoprosthesis on the vessel wall of the bodily vessel. The endoprosthesis according to the invention thus has two different open structures, on the one hand the main structure, which primarily serves to provide the supporting effect and exertion of force on the vessel wall, and on the other hand the carrier structure, which carries the active ingredient and does not act as a mechanical vessel support. The carrier structure consists here of the same material as the main structure and is connected thereto, preferably in a form-fitting manner. The main structure and carrier structure are formed integrally and are preferably produced in one process step.

The individual elements or carriers of the main structure of an intraluminal endoprosthesis, in particular of a stent, usually have a rectangular cross section. With implantation of the endoprosthesis in the bodily vessel, one side of this rectangular cross section comes into contact with the vessel wall. The endoprosthesis is pressed or pushed against the vessel wall during implantation. The side of the rectangular cross section of the strut or of the element of the main structure forming the outer lateral side of the cylindrical endoprosthesis is pushed against the vessel wall. This side is referred to as the abluminal side or as the abluminal portion of the carrier or of the element. The other three sides of the rectangular cross section accordingly do not contact the vessel wall in the lumen of the bodily vessel and are referred to as the luminal side or as the luminal portion of the strut or of the element.

The main difference between main structure and carrier structure lies in the mechanical supporting effect, as already mentioned. Accordingly, the carrier structure also has individual struts, wherein the struts of the carrier structure in at least one dimension are much smaller than the struts of the main structure. By way of example, in the case of a rectangular cross section of the struts of the carrier structure, the length of the two sides perpendicular to the abluminal side of the cross section is preferably less than 75 % of the corresponding sides of the struts of the main structure, particularly preferably less than 50 % and more preferably less than 25 %.

With an endoprosthesis according to the prior art, there are two possibilities for the application of the active ingredient. Either the active ingredient is arranged only on the abluminal side of the carrier or elements of the endoprosthesis, or all sides of the carrier or elements of the endoprosthesis are provided with active ingredient. The first possibility of only coating the abluminal sides is complex in terms of the method. The second possibility means that the active ingredient is transported quickly away from the site of implantation from the luminal sides of the carrier or elements of the endoprostheses and is accumulated in the organism.

The endoprosthesis of the present invention overcomes these disadvantages of the prior art in that the endoprosthesis according to the invention has a carrier structure for the active ingredient, which is arranged in a zone of abated flow of the endoprosthesis. Due to the generation of an area of abated flow by the main structure and the carrier structure, the active ingredient introduced in this area is transported away much more slowly. Accordingly, much higher active ingredient concentrations can be achieved at the site of implantation, whereby the efficacy of the active ingredient entry is increased and the risk of thrombus formation and of restenosis is considerably minimized.

The invention makes use of the knowledge that the bodily fluid does not flow over or through the endoprosthesis uniformly. With the implantation of a stent in an artery, areas of abated flow for example are formed in front of the individual elements of the main structure. Blood flows over these elements, similarly to a weir in the flow, wherein part of the flow is also reflected here, such that areas of abated flow form before. The carrier structure for the active ingredient can be arranged in such areas, for example, such that the active ingredient is transported away much more slowly through the blood and remains longer in higher concentration at the site of implantation.

The endoprosthesis according to the invention is configured such that the areas of abated flow are produced when the cylindrical form of the main body, formed by the main structure, are retained with the implantation. In other words, the areas of abated flow are configured for the fact that a fluid will flow through the cylindrical main structure. Here, the entire structure of the endoprosthesis is to be considered. The endoprosthesis according to the invention has a carrier structure in the area of abated flow. Here, the area of abated flow does not necessarily have to be formed merely by the main structure. A carrier structure is arranged decisively in the area of abated flow. In other words, the flow abatement at the carrier structure can be caused by the main structure and/or the carrier structure itself.

The main structure advantageously consists of at least two meander-shaped segments, and at least one carrier structure is arranged at a maximum and/or minimum of a meander-shaped segment. Here, it is particularly advantageous when the carrier structure is arranged between two adjacent maxima and/or two adjacent minima of a meander-shaped segment, and in particular interconnects the adjacent maxima and/or minima in regions.

Within the scope of the invention, the term "meander-shaped" is to be understood to mean all structures that change direction in an alternating manner. These may be zigzag shapes, sine curves, sawtooth shapes or rectangle shapes.

A main structure formed of at least meander-shaped segments is established in the prior art and is tested in respect of the mechanical supporting effect on the vessel wall of the bodily vessel. The previously discussed flow conditions, similarly to a weir, occur particularly clearly in the maxima and minima of the meander-shaped segments of the main structure. The maxima and minima of the meander-shaped segments of the main structure are oriented from the first end to the second end with respect to the orientation of the longitudinal axis of the main body of the endoprosthesis.

The flow conditions in the endoprosthesis are complex and dependent on the bodily vessel and the respective medium flowing through. A main structure formed of meander-shaped segments always has parts of the segments with a component in the longitudinal direction and a component transverse to the longitudinal direction of the endoprosthesis. Accordingly, segments with components transversely and longitudinally to the flow direction are also always provided. The respective medium therefore inevitably always flows past or over the areas between the maxima and minima in part, such that areas of abated flow are produced between the maxima and minima of the meander-shaped segments. At least one carrier structure for the active ingredient is advantageously arranged in these areas.

In one embodiment of the invention, at least two adjacent meander-shaped segments are arranged in such a way that at least one maximum of the first meander-shaped segment is followed by a minimum of the second meander-shaped segment with respect to the direction of the longitudinal axis of the main body from the first to the second end. In this embodiment of the invention, the main structure of the endoprosthesis consists of at least two meander-shaped segments that are arranged in a manner almost mirrored relative to one another. With an arrangement of this type, a maximum of one segment is always opposite a minimum of the other segment.

In another embodiment of the invention, at least two adjacent meander-shaped segments are arranged in such a way that a maximum of the first meander-shaped segment is followed by a maximum of the second meander-shaped segment with respect to the direction of the longitudinal axis of the main body from the first to the second end. This embodiment of the invention differs from the previously described embodiment in that, here, the meander-shaped segments of the main structure are oriented identically. The meander-shaped segments are not arranged almost in a mirrored manner, but in series.

In addition to the mirrored and series arrangement, the maxima and minima of successive, meander-shaped segments can also be slightly phase-shifted. Such embodiments with a slight phase shift are also understood within the scope of the invention as an arrangement in series or mirrored.

The main structure advantageously consists of at least two meander-shaped ring segments, which are interconnected. It is also advantageous that the main structure consists of at least two meander-shaped segments arranged in a spiraled manner, which are interconnected. In this embodiment of the invention, the areas of abated flow would be produced substantially by the carrier structure arranged in accordance with the invention. Here, the segments of the endoprosthesis arranged both annularly and in a spiraled manner can be arranged in a mirrored manner or in series, as discussed beforehand. Combinations of segments arranged in a mirrored manner or in series may also prove to be advantageous in certain embodiments of the invention.

The carrier structure between two adjacent maxima and/or minima of a segment is preferably honeycomb-shaped. Honeycomb-shaped or mesh-shaped elements lead in particular to the deceleration of the flow and to the formation of recirculation zones between the maxima and/or minima of the segments. Lattice-shaped arrangements are also advantageous. An individual strut will be expedient as a carrier structure.

In a preferred embodiment of the invention, the main structure of the endoprosthesis consists of a number of meander-shaped segments arranged annularly, which are interconnected. The individual meander-shaped segments are arranged here in a mirrored manner. In other words, a maximum of one meander-shaped segment is followed by a minimum of the next adjacent segment with respect to the longitudinal axis of the cylindrical endoprosthesis. It has been found that the effects in this embodiment of the invention of a medium flowing over and past these elements are particularly large. Here, areas of abated flow are produced in particular within the maxima and minima and primarily at the points at a short distance between the segments. The arrangement of the carrier structure between the minima and maxima is therefore particularly advantageous.

The carrier structure is formed from at least one strut, and the main structure is preferably formed from at least two elements, wherein the struts have a shorter height than the elements of the main structure. In particular, the height of the elements of the main structure to the height of the struts of the carrier structure forms a ratio of more than 1.5:1, preferably more than 2:1, and in particular the elements have a height of 500 µm to 50 µm, preferably 80 µm. The height of the elements of the main structure and therefore the height of the struts of the carrier structure are dependent on the desired application of the endoprosthesis. With use of the endoprosthesis as a stent in the coronary area, the elements advantageously have a height of 80 µm. With a use as a peripheral stent, greater heights from 200 µm up to 250 µm are more expedient.

Within the scope of this invention, the height is understood to mean the extension of the struts or elements in the radial direction.

With an embodiment of the invention with a honeycomb-shaped or mesh-shaped carrier structure, the ratio of the mesh width of the carrier structure to the height of the struts is at most 5:1. With a honeycomb-shaped or lattice-shaped carrier structure, the carrier structure is composed of a number of individual cells. The minimal mesh width is understood to mean the smallest distance between two struts of a cell.

Within the scope of this invention, the mesh width is understood to mean the removal of the individual struts of the carrier structure with identical orientation.

The carrier structure is advantageously coated directly with the active ingredient, has reservoirs containing active ingredient and/or has a coating which contains the therapeutic active ingredient.

The main structure and/or the carrier structure expediently consist of biocompatible metal, in particular a cobalt-base alloy, preferably a biologically degradable metal, in particular a magnesium alloy, or a shape-memory material, in particular nitinol, or a polymer. The main structure and carrier structure are preferably made of the same material. In some embodiments of the invention, however, it is expedient to use a material combination for the main structure and carrier structure, in particular a metal material for the main structure and a polymer, which in particular is biologically degradable, for the carrier structure.

The main structure is advantageously coated directly with the active ingredient, has reservoirs containing active ingredient and/or has a coating which contains the therapeutic active ingredient. In this embodiment of the invention, the entire main structure is also used as carrier for the active ingredient. This embodiment of the invention is characterized by particularly simple production, for example by means of a dipping or spraying method.

Due to the present invention, it is possible in particular to maintain the concentration of the active ingredient at the site of implantation for a longer period of time. It is possible with the present invention to selectively adapt the active ingredient release to the extension of the stenosis by corresponding positioning of the carrier structure.

The invention will be explained in greater detail hereinafter on the basis of the exemplary embodiment illustrated in the figure.

In the figure:

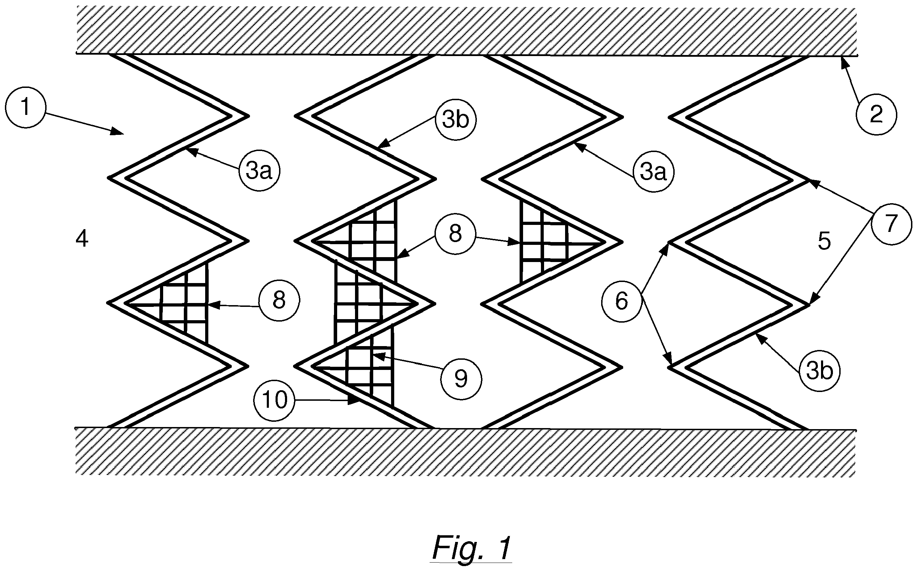

- Fig. 1: shows an exemplary embodiment of the invention with a main structure formed of meander-shaped segments arranged in a mirrored manner.

Figure 1 shows an exemplary embodiment of a stent 1 according to the invention implanted in a bodily vessel 2. The main structure of the stent 1 consists of a number of meander-shaped ring segments 3a and 3b, which are interconnected (not illustrated). In this embodiment of the invention, the ring segments 3a, 3b are configured in a zigzag shape and are arranged in such a way that a maximum 7 of a ring segment 3a is in each case opposite a minimum 6 of the adjacent ring segment 3b. The maxima 7 and minima 6 of the ring segments 3a, 3b are opposite one another here with respect to the longitudinal axis (not illustrated) of the stent 1, said longitudinal axis extending from the first end 4 to the second end 5 of the stent.

A carrier structure 8 for the active ingredient is arranged in some of the maxima 7 and minima 6 of the ring segments 3a, 3b. In fluidic simulations, it has been found that areas of abated flow (recirculation zones) form between the maxima 7 and minima 6. In accordance with the invention, the carrier structure 8 for the active ingredient is arranged in these areas. In this exemplary embodiment of the invention, the carrier structure 8 is formed as a lattice structure.

The height of the struts 9 of the carrier structure is smaller here than the height of the elements 10 of the ring segments 3a, 3b and is 100 µm. The ratio of the height of the elements 10 to the height of the struts 9 is 2:1.

## Claims

1. An intraluminal endoprosthesis (1) suitable for implantation in a bodily vessel (2) through which a fluid flows and having a cylindrical main body with a first end (4) and a second end (5), wherein the main body consists of a main structure (3a, 3b), which is suitable for attaining a mechanical supporting effect in the bodily vessel (2), wherein at least one additional carrier structure (8) is connected to the main structure (3a, 3b), the carrier structure carries at least one therapeutic active ingredient, is connected to the main structure (3a, 3b) in such a way that the carrier structure (8) is located in an area of abated flow when the respective medium flows through the main body from the first end (4) thereof in the direction of the second end (5) thereof, **characterised in that** the main structure (3a, 3b) and carrier structure (8) are formed integrally and the carrier structure (8) consists of the same material as the main structure.

2. The endoprosthesis according to Claim 1, **characterized in that** the main structure consists of at least two meander-shaped segments (3a, 3b) and at least one carrier structure (8) is arranged at a maximum (7) and/or minimum (6) of a meander-shaped segment (3a, 3b).

3. The endoprosthesis according to Claim 2, **characterized in that** the carrier structure (8) is arranged between two adjacent maxima (7) and/or two adjacent minima (6) of a meander-shaped segment (3a, 3b), and in particular interconnects the adjacent maxima (7) and/or the minima (6) in regions.

4. The endoprosthesis (1) according to Claim 2 or 3, **characterized in that** at least two adjacent meander-shaped segments (3a, 3b) are arranged in such a way that at least one maximum (7) of the first meander-shaped segment (3a) is followed by a minimum (6) of the second meander-shaped segment (3b) with respect to the direction of the longitudinal axis of the main body from the first (4) to the second end (5).

5. The endoprosthesis (1) according to Claim 2 or 3, **characterized in that** at least two adjacent meander-shaped segments (3a, 3b) are arranged in such a way that at least one maximum (7) of the first meander-shaped segment (3a) is followed by a maximum (7) of the second meander-shaped segment (3b) with respect to the direction of the longitudinal axis of the main body from the first (4) to the second end (5).

6. The endoprosthesis (1) according to Claim 4 or 5, **characterized in that** the main structure consists of at least two meander-shaped ring segments (3a, 3b), which are interconnected.

7. The endoprosthesis according to Claim 4 or 5, **characterized in that** the main structure consists of at least two meander-shaped segments arranged in a spiraled manner, which are interconnected.

8. The endoprosthesis (1) according to one of the preceding claims, **characterized in that** the carrier structure (8) between two adjacent maxima (7) and/or minima (6) of a segment (3a, 3b) is honeycomb-shaped or mesh-shaped.

9. The endoprosthesis (1) according to one of the preceding claims, **characterized in that** the carrier structure is formed from at least one strut (9) and the main structure (3a, 3b) is formed from at least two elements (10), wherein the struts (9) have a shorter height than the elements (10).

10. The endoprosthesis according to Claim 9, **characterized in that** the ratio of the height of the elements (10) to the height of the struts (9) is greater than 1.5:1, preferably greater than 2:1, in particular the elements (10) have a height between 500 µm and 50 µm, preferably 80 µm.

11. The endoprosthesis according to one of the preceding claims, **characterized in that** the carrier structure (8) is coated directly with the active ingredient, has reservoirs containing active ingredient and/or has a coating which contains the therapeutic active ingredient.

12. The endoprosthesis (1) according to one of the preceding claims, **characterized in that** the main structure (3a, 3b) and the carrier structure (8) consist of a biocompatible metal, in particular a cobalt-base alloy, preferably a biologically degradable metal, in particular a magnesium alloy, or a shape-memory material, in particular nitinol, or a polymer.

13. The endoprosthesis (1) according to one of the preceding claims, **characterized in that** the main structure (3a, 3b) is directly coated with the active ingredient, has reservoirs containing active ingredient and/or has a coating which contains the therapeutic active ingredient.

14. The endoprosthesis (1) according to one of the preceding claims, **characterized in that** the main structure (3a, 3b) and carrier structure (8) consist of different materials.

## Patentansprüche

1. Intraluminale Endoprothese (1), die zur Implantation in ein Körpergefäß (2), durch das ein Fluid strömt, geeignet ist und die einen zylindrischen Hauptkörper mit einem ersten Ende (4) und einem zweiten Ende (5) aufweist, wobei der Hauptkörper aus einem Hauptgebilde (3a, 3b) besteht, das zum Erhalten einer mechanischen Stützwirkung in dem Körpergefäß (2) geeignet ist,
wobei mindestens ein zusätzliches Trägergebilde (8) mit dem Hauptgebilde (3a, 3b) verbunden ist, das Trägergebilde mindestens einen therapeutischen Wirkstoff trägt und mit dem Hauptgebilde (3a, 3b) derart verbunden ist, dass das Trägergebilde (8) sich in einem Bereich verminderter Strömung befindet, wenn das jeweilige Medium durch den Hauptkörper von dem ersten Ende (4) davon in der Richtung des zweiten Endes (5) davon strömt, **dadurch gekennzeichnet, dass** das Hauptgebilde (3a, 3b) und das Trägergebilde (8) integral gebildet sind und das Trägergebilde (8) aus demselben Material wie das Hauptgebilde besteht.

2. Endoprothese (1) nach Anspruch 1, **dadurch gekennzeichnet, dass** das Hauptgebilde aus mindestens zwei meanderförmigen Segmenten (3a, 3b) besteht und mindestens ein Trägergebilde (8) an einem Höhepunkt (7) und/oder Tiefpunkt (6) eines meanderförmigen Segments (3a, 3b) angeordnet ist.

3. Endoprothese nach Anspruch 2, **dadurch gekennzeichnet, dass** das Trägergebilde (8) zwischen zwei nebeneinanderliegenden Höhepunkten (7) und/oder zwei nebeneinanderliegenden Tiefpunkten (6) des meanderförmigen Segment (3a, 3b) angeordnet ist und insbesondere die nebeneinanderliegenden Höhepunkte (7) und/oder Tiefpunkte (6) in Regionen miteinander verbindet.

4. Endoprothese (1) nach Anspruch 2 oder 3, **dadurch gekennzeichnet, dass** mindestens zwei nebeneinanderliegende meanderförmige Segmente (3a, 3b) derart angeordnet sind, dass auf mindestens einen Höhepunkt (7) des ersten meanderförmigen Segments (3a) ein Tiefpunkt (6) des zweiten meanderförmigen Segments (3b) mit Bezug auf die Richtung der Längsachse des Hauptkörpers vom ersten (4) zum zweiten Ende (5) folgt.

5. Endoprothese (1) nach Anspruch 2 oder 3, **dadurch gekennzeichnet, dass** mindestens zwei nebeneinanderliegende meanderförmige Segmente (3a, 3b) derart angeordnet sind, dass auf mindestens einen Höhepunkt (7) des ersten meanderförmigen Segments (3a) ein Höhepunkt (7) des zweiten meanderförmigen Segments (3b) mit Bezug auf die Richtung der Längsachse des Hauptkörpers vom ersten (4) zum zweiten Ende (5) folgt.

6. Endoprothese (1) nach Anspruch 4 oder 5, **dadurch gekennzeichnet, dass** das Hauptgefüge aus mindestens zwei meanderförmigen Ringsegmenten (3a, 3b) besteht, die miteinander verbunden sind.

7. Endoprothese nach Anspruch 4 oder 5, **dadurch gekennzeichnet, dass** das Hauptgefüge aus mindestens zwei meanderförmigen Segmenten besteht, die auf spiralförmige Weise angeordnet und die miteinander verbunden sind.

8. Endoprothese (1) nach einem der vorhergehenden Ansprüche, **dadurch gekennzeichnet, dass** das Trägergefüge (8) zwischen zwei nebeneinanderliegenden Höhepunkten (7) und/oder Tiefpunkten (6) eines Segments (3a, 3b) honigwabenförmig oder netzartig ist.

9. Endoprothese (1) nach einem der vorhergehenden Ansprüche, **dadurch gekennzeichnet, dass** das Trägergefüge aus mindestens einer Strebe (9) gebildet ist und das Hauptgefüge (3a, 3b) aus mindestens zwei Elementen (10) gebildet ist, wobei die Streben (9) eine kürzere Höhe aufweisen als die Elemente (10).

10. Endoprothese nach Anspruch 9, **dadurch gekennzeichnet, dass** das Verhältnis der Höhe der Elemente (10) zu der Höhe der Streben (9) höher als 1,5;1, bevorzugt höher als 2:1, ist, und die Elemente (10) insbesondere eine Höhe zwischen 500 µm und 50 µm, bevorzugt 80 µm aufweisen.

11. Endoprothese nach einem der vorhergehenden Ansprüche, **dadurch gekennzeichnet, dass** das Trägergefüge (8) direkt mit dem Wirkstoff beschichtet ist, Reservoire, die Wirkstoff enthalten, und/oder eine Beschichtung, die den therapeutischen Wirkstoff enthält, aufweist.

12. Endoprothese (1) nach einem der vorhergehenden Ansprüche, **dadurch gekennzeichnet, dass** das Hauptgebilde (3a, 3b) und das Trägergebilde (8) aus einem biokompatible Metall, insbesondere einer Legierung auf der Basis von Kobalt, bevorzugt einem biologisch abbaubaren Metall, insbesondere einer Magnesiumlegierung oder einem Formgedächtnismaterial, insbesondere Nitinol, oder einem Polymer besteht.

13. Endoprothese (1) nach einem der vorhergehenden Ansprüche, **dadurch gekennzeichnet, dass** das Hauptgebilde (3a, 3b) direkt mit dem Wirkstoff beschichtet ist, Reservoire, die Wirkstoff enthalten, und/oder eine Beschichtung, die den therapeutischen Wirkstoff enthält, aufweist.

14. Endoprothese (1) nach einem der vorhergehenden Ansprüche, **dadurch gekennzeichnet, dass** das Hauptgefüge (3a, 3b) und Trägergefüge (8) aus verschiedenen Materialien bestehen.

## Revendications

1. Endoprothèse intraluminale (1) appropriée pour une implantation dans un vaisseau du corps (2) par lequel un fluide s'écoule et ayant un corps principal cylindrique avec une première extrémité (4) et une seconde extrémité (5), où le corps principal est constitué d'une structure principale (3a, 3b) qui est appropriée pour avoir un effet de support mécanique dans le vaisseau du corps (2), où au moins une structure de support (8) supplémentaire est connectée à la structure principale (3a, 3b), la structure de support porte au moins un ingrédient actif thérapeutique, est connectée à la structure principale (3a, 3b) de telle façon que la structure de support (8) est localisée dans une zone d'écoulement réduit lorsque le milieu respectif s'écoule à travers le corps principal de la première extrémité (4) de celui-ci en direction de la seconde extrémité (5) de celui-ci, **caractérisée en ce que** la structure principale (3a, 3b) et la structure de support (8) sont formées d'un seul tenant et la structure de support (8) est constituée du même matériau que la structure principale.

2. Endoprothèse selon la revendication 1, **caractérisée en ce que** la structure principale est constituée d'au moins deux segments en forme de méandres (3a, 3b) et au moins une structure de support (8) est agencée au niveau d'un maximum (7) et/ou d'un minimum (6) d'un segment en forme de méandres (3a, 3b).

3. Endoprothèse selon la revendication 2, **caractérisée en ce que** la structure de support (8) est agencée entre deux maxima adjacents (7) et/ou deux minima adjacents (6) du segment en forme de méandres (3a, 3b) et en particulier interconnecte les maxima (7) et/ou les minima (6) adjacents par endroits.

4. Endoprothèse (1) selon la revendication 2 ou 3, **caractérisée en ce qu'**au moins deux segments en forme de méandres (3a, 3b) sont agencés de telle manière qu'au moins un maximum (7) du premier segment en forme de méandres (3a) est suivi d'un minimum (6) d'un deuxième segment en forme de méandres (3b) par rapport à la direction de l'axe longitudinal du corps principal à partir de la première extrémité (4) à la seconde (5).

5. Endoprothèse (1) selon la revendication 2 ou 3, **caractérisée en ce qu'**au moins deux segments en forme de méandres (3a, 3b) sont agencés de telle manière qu'au moins un maximum (7) du premier segment en forme de méandres (3a) est suivi par un maximum (7) du deuxième segment en forme de méandres (3b) par rapport à la direction de l'axe longitudinal du corps principal à partir de la première extrémité (4) vers la seconde extrémité (5).

6. Endoprothèse (1) selon la revendication 4 ou 5, **caractérisée en ce que** la structure principale est constituée d'au moins deux segments annulaires en forme de méandres (3a, 3b) qui sont interconnectés.

7. Endoprothèse selon la revendication 4 ou 5, **caractérisée en ce que** la structure principale est constituée d'au moins deux segments en forme de méandres, agencés à la manière d'une spirale, qui sont interconnectés.

8. Endoprothèse (1) selon l'une des revendications précédentes, **caractérisée en ce que** la structure de support (8) entre deux maxima (7) et/ou minima (6) adjacents d'un segment (3a, b) est en forme de rayon de miel ou en forme de maille.

9. Endoprothèse (1) selon l'une des revendications précédentes, **caractérisée en ce que** la structure de support est formée à partir d'au moins un étai (9) et la structure principale (3a, 3b) est formée à partir d'au moins deux éléments (10), où les étais (9) ont une hauteur inférieure aux éléments (10).

10. Endoprothèse selon la revendication 9, **caractérisée en ce que** le rapport de la hauteur des éléments (10) sur la hauteur des étais (9) est supérieur à 1,5:1, de préférence supérieur à 2:1, en particulier, les éléments (10) ont une hauteur entre 500 µm et 50 µm, de préférence de 80 µm.

11. Endoprothèse selon l'une des revendications précédentes, **caractérisée en ce que** la structure de support (8) est revêtue directement avec l'ingrédient actif, a des réservoirs contenant l'ingrédient actif, et/ou a un revêtement qui contient l'ingrédient actif thérapeutique.

12. Endoprothèse (1) selon l'une des revendications précédentes, **caractérisée en ce que** la structure principale (3a, 3b) et la structure de support (8) sont constituées d'un métal biocompatible, en particulier d'un alliage à base de cobalt, de préférence, d'un métal dégradable biologiquement, en particulier, d'un alliage de magnésium, ou d'un matériau à mémoire de forme, en particulier du nitinol ou un polymère.

13. Endoprothèse (1) selon l'une des revendications précédentes, **caractérisée en ce que** la structure principale (3a, 3b) est directement revêtue avec l'ingrédient actif, a des réservoirs contenant l'ingrédient actif, et/ou a un revêtement qui contient l'ingrédient actif thérapeutique.

14. Endoprothèse (1) selon l'une des revendications précédentes, **caractérisée en ce que** la structure principale (3a, 3b) et la structure de support (8) sont constituées de matériaux différents.
